# FASCICULE DE BREVET EUROPEEN

(11) **EP 0 547 931 B1**
(45) Date de publication et mention de la délivrance du brevet: **05.03.1997**
(21) Numéro de dépôt: 92403226.1
(22) Date de dépôt: 30.11.1992
(51) Int. Cl.: A61B 3/113

(54) **Procédé et dispositif de mesure des mouvements oculaires**
Verfahren und Gerät zur Messung der Augenbewegungen
Process and apparatus to measure the movements of the eyes

(30) Priorité: 02.12.1991 FR 9114901
(43) Date de publication de la demande: 23.06.1993
(73) Titulaire: COMMISSARIAT A L'ENERGIE ATOMIQUE, 75015 Paris (FR)
(72) Inventeur: Masse, Dominique, F-38500 Coublevie (FR); Baudoin, Christian, F-38470 Vinay (FR)
(74) Mandataire: Signore, Robert

(56) Documents cités:
- EP-A- 0 286 490
- WO-A-86/03113
- GB-A- 2 207 748
- US-A- 4 958 925

## Description

La présente invention concerne un procédé et son dispositif de mise en oeuvre, pour mesurer les mouvements des yeux et ainsi connaître, par exemple, la direction du regard.

Elle trouve des applications dans des domaines nombreux et variés. En effet, dans le domaine médical, elle permet l'aide au diagnostic, notamment en ophtalmologie pour diagnostiquer le strabisme, en O.R.L. pour les troubles de l'équilibre et en neurologie. L'invention peut aussi s'appliquer à la recherche scientifique sur la physiologie du système visuel et du système vestibulaire, ou encore, à l'étude sur le mal des transports dont, en particulier, le mal de l'espace. Une autre application de l'invention se rapporte à l'ergonomie et, plus particulièrement, à l'analyse de la stratégie de prise d'information visuelle et de la fatigue sur un poste de travail.

L'invention peut s'appliquer à d'autres domaines encore, tels que l'aide aux handicapés tétraplégiques, l'étude des mécanismes de la lecture et la publicité.

L'oeil est, en effet, un organe de l'homme qui suscite de nombreuses études.

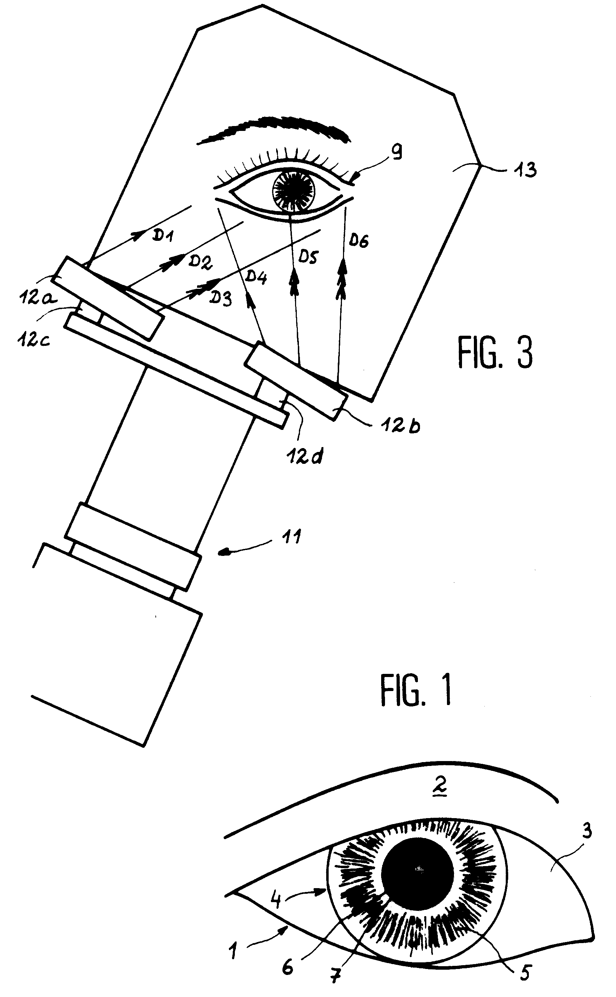

Sur la figure 1, on a représenté schématiquement un oeil vu de face. Sur cette figure, on a représenté l'orbite 1 de l'oeil au-dessus de laquelle se trouve la paupière 2. Dans l'orbite 1, on peut voir une partie de la sclérotique 3 qui forme une sorte de sphère blanche, présentant une ouverture circulaire où est insérée la cornée 4, un peu plus bombée que la sclérotique 3. La cornée 4 est transparente et permet de discerner, par transparence, l'iris 5 qui est une membrane opaque percée d'une ouverture circulaire, à savoir la pupille 6. Sur cette figure, on a représenté, également, le reflet cornéen 7, c'est-à-dire le reflet que l'on peut distinguer sur la cornée 4 lorsque l'oeil est éclairé.

Mesurer le mouvement d'un oeil consiste, de façon habituelle, à déterminer la position de la pupille et du reflet cornéen de l'oeil. Cependant, lorsque l'oeil est éclairé sans précautions particulières, les images réalisées peuvent contenir certaines parties sombres, comme les cils et les sourcils, qui se confondent avec le disque sombre de la pupille. De même, certaines parties claires de l'image réalisée, telles que les reflets sur la peau des paupières ou le blanc de la sclérotique, peuvent être confondues avec la tâche brillante du reflet cornéen. Ces parties respectivement sombres ou claires faussent le calcul du centre, respectivement, de la pupille ou du reflet cornéen.

Aussi, certains procédés ont été réalisés par des hommes de l'art pour remédier à ces inconvénients. Certains de ces procédés sont décrits ci-dessous.

Un procédé photographique, pour mesurer ces mouvements des yeux, consiste à photographier (ou filmer) l'oeil. Les images obtenues sur le film photographique (ou vidéo) sont exploitées après un dépouillement fastidieux. Ce procédé est non seulement assez fastidieux, mais il n'est, de plus, pas exploitable en temps réel.

Un procédé photoélectrique mis en oeuvre par le dispositif nommé OBER² et décrit dans l'article "Eye movement registration system" de la revue "Pre-released information" de Mai 1988, consiste à éclairer l'oeil par une source infrarouge. Une photodiode, placée près de l'oeil, mesure la quantité de lumière réfléchie globalement par le globe oculaire. L'iris et la pupille de l'oeil renvoyant moins de lumière que la sclérotique blanche, il est assez simple d'en déduire globalement le mouvement de l'oeil. Cependant, si ce procédé est relativement simple à mettre en oeuvre, il n'est guère précis car les éventuels mouvements de paupières faussent la quantité de lumière mesurée par la photodiode.

Un autre procédé, mis en oeuvre notamment par le dispositif nommé "DORNIER eye tracking system" et décrit dans une publication de la société DORNIER, consiste à réaliser des images vidéo de l'oeil et à traiter ces images. Selon ce procédé, l'oeil est éclairé par une lumière infrarouge et filmé par une caméra vidéo. Les images obtenues sont traitées en temps réel par des opérateurs électroniques spécifiques. Ce procédé est précis, mais les opérateurs électroniques sont généralement complexes et d'un coût élevé.

Un autre procédé et un système correspondant sont connus de GB-A-2 207 748

La présente invention a non seulement pour avantage de ne nécessiter qu'une électronique de traitement de l'image relativement simple, mais elle permet, de plus, une amélioration de la qualité des images à traiter (par rapport aux images obtenues selon le procédé précédent), et donc une meilleure précision dans la mesure des mouvements de l'oeil.

L'invention permet, en effet, de mesurer les mouvements horizontaux et verticaux de l'oeil, par rapport à la tête, à partir d'une ou de plusieurs image(s) vidéo corrigée(s) de la plupart des reflets et ombres dûs aux éclairages de prise de vue.

De façon plus précise, l'invention concerne un procédé de mesure des mouvements d'un oeil comportant les étapes suivantes :
- éclairage de l'oeil selon au moins deux directions distinctes ;
- acquisition d'au moins deux images de l'oeil ainsi éclairé ;
- lorsque ces images de l'oeil ont été acquises :
   . conversion de chaque image acquise en une image binaire ;
   . réalisation d'une image corrigée de tous reflets relatifs à l'éclairage sur l'oeil, par combinaison des images binaires ;
- détermination, sur l'image corrigée, de la position de l'oeil par calcul du centre de gravité dudit oeil.

Avantageusement, l'étape d'éclairage de l'oeil consiste en ce que l'oeil éclairé alternativement suivant l'une et l'autre des directions, une image de l'oeil étant alors acquise pour chacun des éclairages.

Appliqué à la mesure de position de la pupille de l'oeil, le procédé consiste à éclairer l'oeil selon une première intensité lumineuse, l'étape de réalisation de l'image corrigée, sur laquelle est mesurée la position de la pupille, consistant à superposer les images obtenues pour chacun des éclairages.

Appliqué à la mesure de position du reflet cornéen de l'oeil, le procédé consiste à éclairer l'oeil selon une seconde intensité lumineuse, l'étape de réalisation de l'image corrigée consistant à choisir l'image, dont la direction de l'éclairage est la plus proche angulairement du reflet cornéen.

Appliqué à la fois à la mesure des positions de la pupille et du reflet cornéen, le procédé consiste à éclairer l'oeil selon une séquence d'éclairage dans laquelle l'oeil est éclairé successivement suivant l'une ou l'autre des directions ou suivant les deux.

Le dispositif de mise en oeuvre du procédé comprend les caractéristiques définies dans le revendication 1.

De façon avantageuse, les moyens d'éclairage de l'oeil comportent au moins deux sources lumineuses orientées de façon différentes par rapport à l'oeil, de façon à ce que les reflets sur l'oeil relatifs à l'éclairage selon les directions distinctes, soient dissociés l'un de l'autre.

Ils comportent, en outre, un générateur de courant connecté à chacune des sources lumineuses, auxquelles il délivre un courant d'intensité et/ou de durée variable(s) assurant des éclairages d'intensités lumineuses diverses.

De plus, les sources lumineuses comprennent chacune une pluralité de diodes électroluminescentes synchronisées, chaque diode étant disposée selon une orientation particulière par rapport à l'oeil.

Selon l'invention, les moyens de traitement comportent un convertisseur analogique/numérique pour transformer les images obtenues par les moyens vidéo en images binaires, la détermination des mouvements de l'oeil étant réalisée à partir de la mesure des positions de l'oeil sur ces images binaires.

Pour déterminer la position de la pupille, les moyens de traitement comportent des moyens de combinaison pour réaliser une image corrigée par superposition des images obtenues lors d'éclairages alternés par les sources lumineuses. Ces moyens de combinaison comportent une mémoire connectée à une sortie du convertisseur analogique/numérique et un circuit logique OU, connecté d'une part à la sortie dudit convertisseur, d'autre part à une sortie de la mémoire et fournissant en sortie l'image corrigée.

En outre, le dispositif de l'invention comporte un séquenceur pour synchroniser les moyens vidéo, les moyens d'éclairage de l'oeil et les moyens de traitement.

D'autres avantages et caractéristiques de l'invention ressortiront de la description qui va suivre donnée, à titre illustratif mais non limitatif, en référence aux dessins dans lesquels :
- la figure 1, déjà décrite, représente schématiquement un oeil vu de face ;
- la figure 2 représente schématiquement une vue de profil de la tête du patient avec le dispositif permettant de mesurer les mouvements de ses yeux ;
- la figure 3 représente schématiquement une vue de face de la partie du dispositif située à proximité de l'oeil avec, notamment, les directions lumineuses éclairant l'oeil ;
- la figure 4 représente, sous forme de chronogramme, un exemple de séquence d'éclairage de l'oeil ; et
- la figure 5 représente le schéma fonctionnel du dispositif selon l'invention.

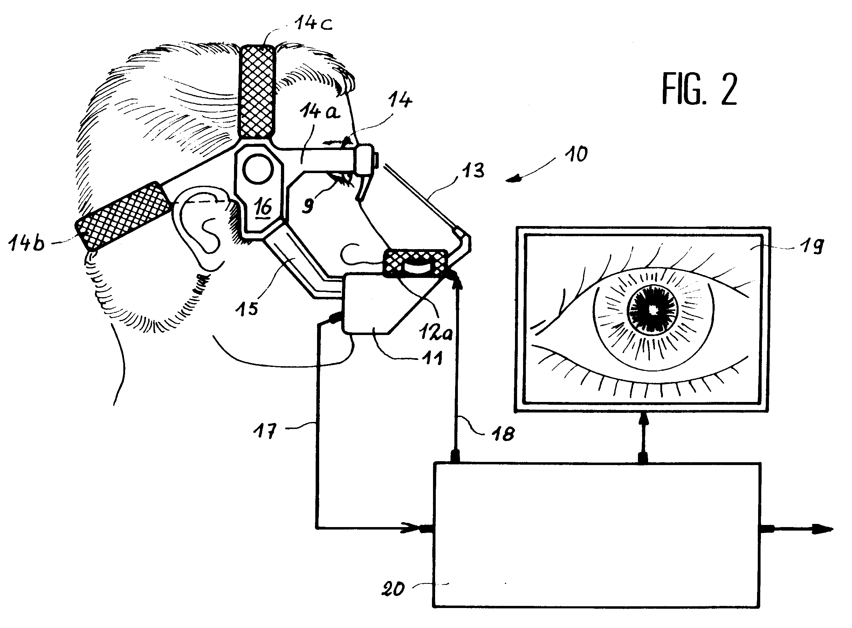

Sur la figure 2, on a représenté globalement le dispositif selon l'invention. Ce dispositif comprend un ensemble 10 de mesure mobile, c'est-à-dire pouvant être disposé à proximité de l'oeil, et des moyens 20 de traitement d'images. L'ensemble 10 de mesure comporte une caméra vidéo miniature 11, des sources lumineuses (dont seule la source 12a est représentée sur la figure) envoyant, suivant des directions différentes, des rayons lumineux sur le miroir 13 incliné de façon à renvoyer la lumière sur l'oeil 9 du patient dont on cherche à déterminer les mouvements oculaires.

Cet ensemble 10 de mesure peut être installé sur une monture de lunette, un casque ou tout autre support adaptable sur la tête du patient.

Selon l'exemple de réalisation de l'invention, l'ensemble 10 de mesure est monté sur une monture de lunette 14. La caméra 11 est fixée sur la monture de lunette 14 par un support de caméra 15. Des moyens 16 de réglage mécaniques permettent de régler la position du support de caméra 15 par rapport à la monture 14 afin de rendre le dispositif adaptable à tout patient. Ces moyens de réglage 16 sont situés sur la monture de lunette 14, plus précisément sur la branche 14a de la monture, les deux branches pouvant être reliées par une ou plusieurs bandes de liaison 14b, 14c, souples, du type sangles, permettant de s'affranchir de la plupart des mouvements résiduels de la monture de lunette 14 par rapport à la tête du patient. En effet, la caméra 11 est réglable en position à partir des moyens de réglage 16 ; les sources lumineuses sont fixées sur la caméra 15, de même que le miroir 13. Aussi, l'ensemble sources lumineuses/miroir/caméra est entièrement solidaire du support de caméra 15 et donc modifiable en position lorsque l'on règle la position du support de caméra 15 par les moyens de réglage 16.

La caméra 11 est reliée électriquement, par la liaison 17, aux moyens 20 de traitement des images. De même, la source lumineuse 12a est reliée par la liaison 18 auxdits moyens 20. En effet, l'image de l'oeil obtenue par la caméra 11 est envoyée aux moyens 20 de traitement qui calculent la position de certains détails de l'oeil, tels que l'iris, la pupille et le reflet cornéen. Ces moyens 20 de traitement permettent de plus de calculer des corrections des images, lors de la détermination de position de la pupille par exemple, et de calculer, sur les positions calculées, des corrections liées à la calibration de l'oeil du patient.

Un écran vidéo 19 est également connecté sur les moyens de traitement 20 afin de permettre la visualisation de l'image de l'oeil.

Selon les applications, un logiciel d'exploitation des mesures est intégré au dispositif.

Le dispositif montré sur la figure 2 permet de mesurer les mouvements horizontaux et verticaux de l'oeil tout en s'affranchissant des mouvements résiduels de la monture de lunette par rapport à la tête. De façon plus précise, il permet, sur une image vidéo corrigée de la plupart des ombres et reflets néfastes, de mesurer le centre de la pupille et du reflet cornéen et, éventuellement, d'observer l'iris.

Il met en oeuvre, effectivement, un procédé basé sur l'optimisation des conditions d'éclairage de l'oeil. En effet, un éclairage optimal de l'oeil permet d'obtenir des images de l'oeil où la pupille apparaît comme un simple disque sombre et où le reflet cornéen apparaît comme une seule tâche brillante.

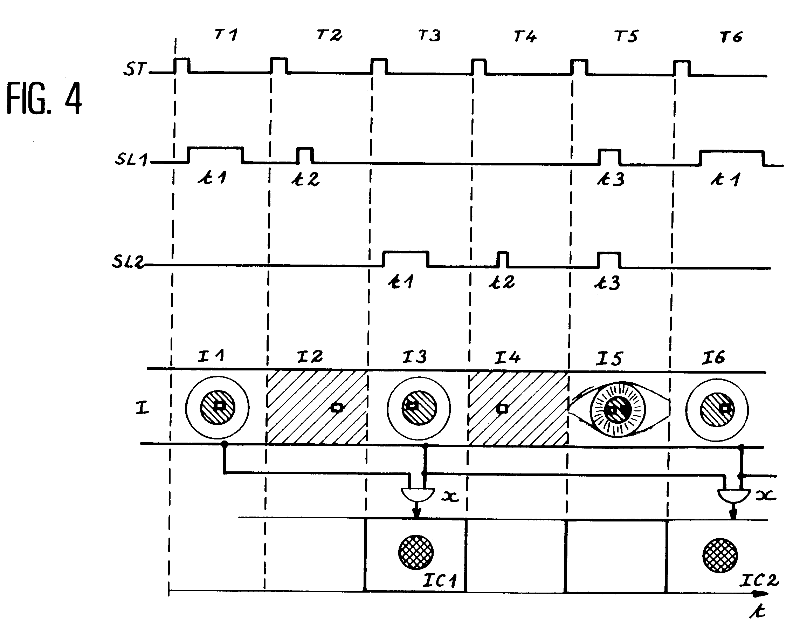

Sur la figure 3, on a représenté, vue de face, la partie du dispositif située à proximité de l'oeil. Cette figure montre donc une partie de la caméra 11 sur laquelle reposent, par l'intermédiaire de deux supports 12c et 12d, les sources lumineuses 12a et 12b. Ces sources lumineuses émettent des rayons lumineux, de façon indirecte, vers l'oeil 9. En effet, les rayons sont émis selon des directions distinctes D1 à D6 vers le miroir 13 qui réfléchit lesdits rayons sur l'oeil 9.

Les sources lumineuses 12a et 12b sont orientées, indépendamment l'une de l'autre, de façon à fournir un éclairage optimal de l'oeil 9. Pour cela, chaque source lumineuse comporte un boîtier dans lequel sont disposées un certain nombre de diodes électroluminescentes (L.E.D.), non représentées sur la figure par mesure de simplification. Ces diodes, généralement au nombre de quatre à dix par boîtier, émettent simultanément de la lumière et sont orientées de façon à optimiser l'éclairage de l'oeil 9. Chaque diode a sa propre orientation ; elle est directive et émet de la lumière dans un cône de demi-angle, à savoir vingt degrés environ.

De façon plus pratique, chaque diode est orientée de façon à diminuer les zones ombrées qui apparaissent initialement sur l'oeil 9 et à compenser la diminution de la réflexion en direction de la caméra 11.

Sur la figure 3, chaque source lumineuse 12a, 12b représentée comporte trois diodes émettant de la lumière suivant les directions respectives D1, D2, D3, D4, D5 et D6.

Sur cette figure, et dans toute la description, il est fait référence à deux sources lumineuses 12a et 12b. Le dispositif peut, bien entendu, comporter trois, quatre ou de nombreuses sources lumineuses, telles que décrites précédemment.

En effet, pour obtenir une image de la pupille et du reflet cornéen de bonne qualité, il peut être intéressant de positionner plusieurs (au moins deux) sources lumineuses réparties autour de l'oeil.

Il est difficile d'optimiser l'éclairage de l'oeil sur une même image pour obtenir simultanément la pupille, le reflet cornéen et les détails de la pupille. Le procédé selon l'invention consiste donc à réaliser successivement une image de la pupille, une image du reflet cornéen et une image de l'iris, l'éclairage étant alors optimisé pour chacune des images.

On comprendra par "éclairage optimisé" l'éclairage le mieux adapté à la détection de l'objet considéré, en l'occurrence les détails de l'oeil, cet éclairage optimisé permettant l'obtention d'une image de qualité dudit objet.

Aussi, l'éclairage de l'oeil s'effectue séquentiellement. Chaque séquence d'éclairage a une durée de plusieurs trames vidéo, chaque trame vidéo correspondant à un éclairage optimisé pour l'un des détails de l'oeil que l'on cherche à mettre en évidence (par exemple : la pupille ou le reflet cornéen).

De plus, pour obtenir une image corrigée de la pupille, c'est-à-dire une image de la pupille sans ombre ou reflet néfaste, on réalise deux images successives de la pupille suivant deux directions d'éclairage différentes. En effet, lorsqu'une source lumineuse éclaire l'oeil, un reflet (dit reflet cornéen) apparaît sur la pupille. Aussi, afin de masquer ce reflet néfaste, le procédé de l'invention propose d'effectuer une seconde image de la pupille :

la première image est réalisée en éclairant l'oeil avec une première source lumineuse ; la seconde image est réalisée en éclairant l'oeil avec une seconde source lumineuse. Ainsi, les deux reflets dûs aux éclairages des deux sources lumineuses sont masqués en superposant les deux images obtenues, le reflet clair sur la première image étant caché par la zone sombre de la pupille sur la seconde image, et inversement.

Cependant, cette superposition des images ne peut cacher entièrement les reflets clairs que lorsque les deux reflets ne sont pas trop proches, c'est-à-dire s'ils n'ont pas de partie commune. Afin d'éviter ces éventuels problèmes, l'utilisation de plusieurs sources lumineuses est recommandée, le choix des sources lumineuses éclairant l'oeil étant effectué de façon à éviter la superposition des reflets clairs.

L'utilisation de plusieurs sources lumineuses permet de déterminer le reflet cornéen quelle que soit sa position sur le dioptre sphérique air-cornée. En effet, lorsque l'oeil tourne d'un angle supérieur à vingt degrés par rapport à la direction principale de la source lumineuse éclairant l'oeil, le reflet ne se forme plus sur la cornée, mais sur la sclérotique et sur la surface concave de raccordement de la cornée avec la sclérotique. Le reflet prend alors un aspect très irrégulier et inutilisable pour un calcul simple et précis de sa position. Pour ne pas limiter le champ de mesure à vingt degrés alors que les déplacements de l'oeil atteignent jusqu'à plus ou moins quarante degrés, on utilise plusieurs sources lumineuses. On éclaire alors successivement l'oeil avec chacune des sources lumineuses et on choisit l'image prise lors de l'éclairage par une source lumineuse située à moins de vingt degrés de l'axe optique de la cornée.

De plus, pour obtenir une image précise de l'iris, sans reflet et ombre néfastes, on éclaire l'oeil simultanément avec au moins deux sources lumineuses.

Aussi, pour obtenir successivement des images de pupille, de reflet cornéen et d'iris, on éclaire l'oeil selon des séquences d'éclairage prédéterminées.

Un chronogramme représentant un exemple de séquence d'éclairage a été représenté sur la figure 4. La ligne ST représente la synchro-trame de la caméra : à chaque trame T1 à T6 correspond un éclairage particulier de l'oeil. La ligne SL1 représente les temps d'éclairage de la première source lumineuse. La ligne SL2 représente les temps d'éclairage fournis par la seconde source lumineuse. Les temps d'éclairage sont variables afin d'optimiser l'éclairage de l'oeil. En I, on a représenté, pour chaque trame, l'image obtenue à partir des éclairages SL1 et SL2.

On a représenté en bas du chronogramme, le circuit logique OU grâce auquel on superpose deux images de pupille représentée en I.

De façon plus précise, on a représenté sur la ligne ST l'impulsion de synchronisation de trame.

Durant la trame T1, la première source lumineuse SL1 émet durant un temps t1 ; la caméra prend alors une première image I1 sur laquelle apparaît la pupille.

La source lumineuse SL1 émet durant un temps t2, pendant la trame T2 ; la caméra reçoit alors la première image I2 du reflet cornéen.

Durant la trame T3, la seconde source lumineuse SL2 envoie une lumière, durant le temps t1, pour permettre la réalisation d'une seconde image I3 de la pupille. Les images I1 et I3 sont combinées, grâce au circuit logique OU, pour donner une image corrigée IC1 de la pupille.

Durant la trame T4, la source lumineuse SL2 éclaire l'oeil, pendant un temps t2, pour permettre la réalisation d'une seconde image I4 du reflet cornéen.

Durant la trame T5, les deux sources lumineuses SL1 et SL2 éclairent simultanément, durant un temps t3, l'oeil pour permettre l'obtention d'une image détaillée I5 de l'iris.

A partir de la trame T6, on recommence la même séquence d'éclairage que celle décrite précédemment, l'image I6 de la pupille étant combinée avec l'image I3 de la séquence d'éclairage précédente.

A partir d'une telle séquence d'éclairage, on réalise trois images respectives de la pupille, du reflet cornéen et de l'iris. Pour déterminer le mouvement de l'oeil, cette séquence d'éclairage est répétée successivement, une mesure de la position du centre de la pupille et du centre du reflet cornéen étant effectuée pour chaque séquence d'éclairage.

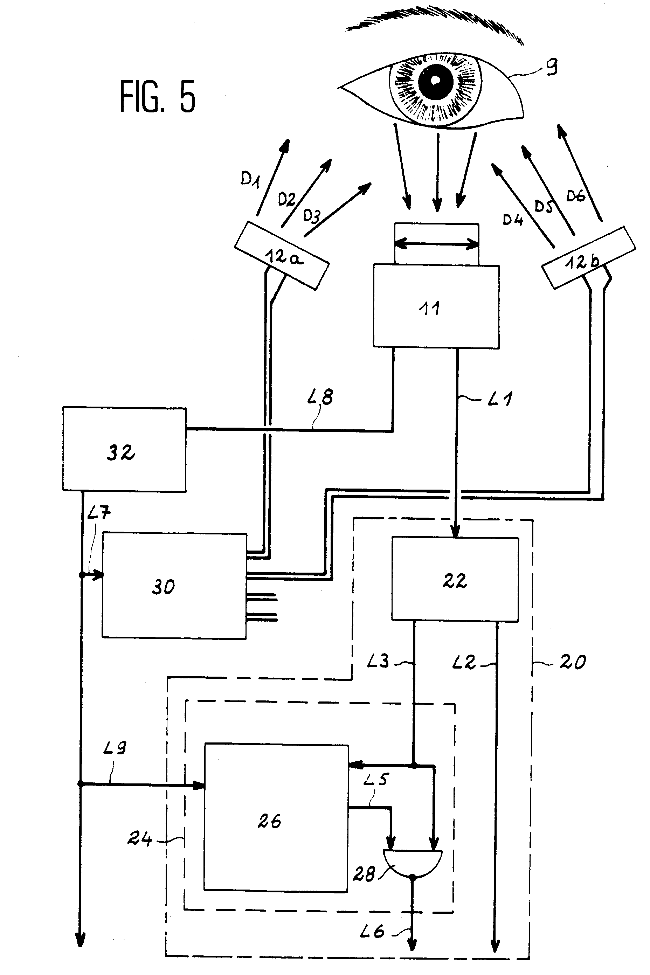

Le dispositif permettant de mettre en oeuvre le procédé de l'invention a été représenté, de façon fonctionnelle, sur la figure 5.

Cette figure montre l'oeil 9 avec les directions d'éclairage D1, D2, D3 provenant de la première source lumineuse 12a et les directions d'éclairage D4, D5, D6 provenant de la seconde source lumineuse 12b. Les images de l'oeil 9 sont réalisées par la caméra 11, puis transmises par la liaison L1 aux moyens de traitement 20.

De façon plus précise, ces moyens de traitement 20 comportent un convertisseur 22 analogique/numérique (A/N) qui transforme l'image analogique reçue de la caméra 11 en une image binaire plus facile à traiter.

Cette image binaire, lorsqu'elle représente le reflet cornéen, est transmise par la liaison L2 vers une carte de calcul du centre du reflet cornéen, non représentée sur la figure.

Lorsque l'image binaire représente la pupille, elle est transmise par la liaison L3 vers des moyens 24 de combinaison de deux images binaires.

Ces moyens 24 de combinaison comportent une mémoire 26 et un circuit logique 28 de fonction OU. L'image binaire sortant du convertisseur A/N 22 est introduite d'une part dans la mémoire 26 et d'autre part dans le circuit logique 28. Une image binaire, introduite précédemment dans la mémoire 26, est transmise parallèlement, par la liaison L5, au circuit logique 28. Généralement, les deux images binaires introduites dans le circuit logique 28 sont l'image de la pupille en cours de traitement et la dernière image de la pupille mémorisée dans la mémoire 26. Ce circuit logique 28 effectue une superposition des deux images binaires introduites. L'image alors obtenue (image corrigée) est transmise par la liaison L6 à une carte de calcul du centre de la pupille, non représentée sur la figure.

Selon un mode de réalisation de l'invention, chaque source lumineuse 12a et 12b est connectée, en entrée, à un générateur 30 de courant. Ce générateur 30 fournit aux sources lumineuses 12a et 12b un courant dont l'intensité est propre à chacune. Ainsi, l'intensité lumineuse fournie par chaque source 12a ou 12b au cours des séquences d'éclairage est modulée pour assurer un éclairage adapté à la bonne détection de l'un des détails de l'oeil (pupille, reflet cornéen, ... ).

Sur la figure 5, on a également représenté le séquenceur 32. Ce séquenceur 32 est connecté par les liaisons respectives L8, L7, L9 à la fois à la caméra 11, au générateur 30 et à la mémoire 26. Il permet de synchroniser, via le générateur 30, les sources lumineuses 12a et 12b avec la caméra 11, et donc chaque éclairage de la séquence d'éclairage avec la trame vidéo correspondante.

Ce séquenceur 32 permet également de synchroniser les moyens 20 de traitement avec la caméra 11 et, plus précisément, de mémoriser dans la mémoire 26, les trames vidéo correspondant à un éclairage de détection de la pupille.

Ce séquenceur 32 permet donc de réaliser les séquences d'éclairage choisies et d'effectuer, sur chacune des images de la séquence, les traitements adéquats à chacune d'elles.

Selon un exemple de réalisation de l'invention, l'oeil dont on cherche à mesurer les mouvements est éclairé par deux sources lumineuses (SL1 et SL2) situées de part et d'autre de l'optique de la caméra. La source SL1 comprend dix diodes électroluminescentes et la source SL2 comprend huit diodes électroluminescentes. Chacune desdites diodes est encastrée dans le boîtier de sa source lumineuse, dans un trou dont la direction a été déterminée expérimentalement. Selon l'exemple, les diodes sont orientées selon des angles variant entre moins vingt degrés et plus vingt degrés par rapport à la normale au boîtier, chaque diode émettant un cône de lumière de demi-angle variant de dix à vingt degrés.

Selon d'autres modes de réalisation de l'invention, d'autres sources lumineuses peuvent être placées de part et d'autre des sources lumineuses 12a et 12b, ou bien placées en avant et en arrière de l'optique de la caméra.

Le procédé selon l'invention, décrit précédemment, peut être mis en oeuvre dans des applications autres que la mesure des mouvements oculaires. Un tel procédé, dans lequel on utilise les sources lumineuses décrites, peut être appliqué pour éliminer des reflets gênants sur différentes sortes d'objets à reconnaître. Il peut également être mis en oeuvre pour accroître la dynamique de codage en niveaux de gris d'une image, ou encore pour éliminer des zones ombrées.

## Revendications

1. Procédé de mesure des mouvements d'un oeil, comportant les étapes suivantes :
- éclairage de l'oeil (9) selon au moins deux directions (D1-D6) distinctes ;
- acquisition d'au moins deux images de l'oeil (I1, I2, I3,...) ainsi éclairé ;
- lorsque ces images de l'oeil ont été acquises :
. conversion de chaque image acquise en une image binaire ;
. réalisation d'une image corrigée (IC1) de tous reflets relatifs à l'éclairage sur l'oeil, par combinaison des images binaires ;
- détermination, sur l'image corrigée, de la position de l'oeil par calcul du centre de gravité dudit oeil.

2. Procédé selon la revendication 1, caractérisé en ce que l'étape d'éclairage de l'oeil consiste en ce que l'oeil est éclairé alternativement suivant l'une et l'autre des directions, une image de l'oeil étant alors acquise pour chacun des éclairages.

3. Procédé selon la revendication 2, dans lequel on réalise une mesure de position de la pupille (6) de l'oeil, caractérisé en ce que l'oeil étant éclairé selon une première intensité lumineuse, l'étape de réalisation de l'image corrigée, sur laquelle est mesurée la position de la pupille, consiste à superposer les images (I1, I3) obtenues pour chacun des éclairages.

4. Procédé selon la revendication 2, dans lequel on réalise une mesure de position du reflet cornéen (7) de l'oeil, caractérisé en ce que l'oeil étant éclairé selon une seconde intensité lumineuse, l'étape de réalisation de l'image corrigée consiste à choisir l'image (I2, I4), dont la direction de l'éclairage est la plus proche angulairement du reflet cornéen.

5. Procédé selon les revendications 3 et 4, dans lequel on réalise à la fois la mesure des positions de la pupille et du reflet cornéen, caractérisé en ce qu'il consiste à éclairer l'oeil selon une séquence d'éclairage dans laquelle l'oeil est éclairé successivement suivant l'une ou l'autre des directions, ou les deux.

6. Dispositif de mesure des mouvements d'un oeil mettant en oeuvre le procédé selon l'une quelconque des revendications 1 à 5, comportant :
- des moyens (12a, 12b) d'éclairage de l'oeil suivant au moins deux directions distinctes ; et
- des moyens (11) vidéo pour réaliser des images de l'oeil à chaque éclairage,
caractérisé en ce qu'il comporte, en outre, des moyens (20) de traitement pour réaliser une image corrigée des reflets dus à chaque éclairage par combinaison desdites au moins deux images et déterminer les mouvements de l'oeil à partir de la position du centre de gravité dudit oeil sur cette image corrigée.

7. Dispositif selon la revendication 6, caractérisé en ce que les moyens d'éclairage de l'oeil comportent au moins deux sources lumineuses orientées de façon différentes par rapport à l'oeil de façon à ce que les reflets sur l'oeil relatifs à l'éclairage selon les directions distinctes, soient dissociés l'un de l'autre.

8. Dispositif selon la revendication 7, caractérisé en ce que les moyens d'éclairage de l'oeil comportent en outre un générateur de courant (30), connecté à chacune des sources lumineuses, auxquelles il délivre un courant d'intensité et/ou de durée variables assurant des éclairages d'intensités lumineuses diverses.

9. Dispositif selon la revendication 7 ou 8, caractérisé en ce que les sources lumineuses comprennent chacune une pluralité de diodes électroluminescentes synchronisées, chaque diode étant disposée selon une orientation particulière par rapport à l'oeil.

10. Dispositif selon l'une quelconque des revendications 6 à 9, caractérisé en ce que les moyens de traitement comportent un convertisseur analogique/numérique (22) pour transformer les images obtenues par les moyens vidéo en images binaires, la mesure des mouvements de l'oeil étant réalisée sur ces images binaires.

11. Dispositif selon la revendication 8, caractérisé en ce que les moyens de traitement comportent des moyens (24) de combinaison pour réaliser une image corrigée par superposition des images obtenues lors d'éclairage alternés par les sources lumineuses.

12. Dispositif selon la revendication 11, caractérisé en ce que les moyens de combinaison comportent une mémoire (26) connectée à une sortie du convertisseur analogique/numérique et un circuit logique OU (28), connecté d'une part à la sortie dudit convertisseur, d'autre part, à une sortie de la mémoire et fournissant en sortie l'image corrigée.

13. Dispositif selon l'une quelconque des revendications 6 à 12, caractérisé en ce qu'il comporte, en outre, un séquenceur (32) pour synchroniser les moyens vidéo, les moyens d'éclairage de l'oeil et les moyens de traitement.

## Claims

1. Method for measuring the movements of an eye, including the following steps:
- illuminating the eye (9) in at least two distinct directions (D1-D6);
- obtaining at least two images of the eye (I1, I2, I3, etc.) thus illuminated;
- when these images of the eye have been obtained:
• converting each image obtained into a binary image;
• creating an image (IC1) corrected for all reflections relative to the illumination on the eye, by combination of the binary images;
- determining, on the corrected image, the position of the eye by calculation of the centre of gravity of the said eye.

2. Method according to Claim 1, characterized in that the step of illuminating the eye consists in the eye being illuminated alternately in one or other of the directions, an image of the eye then being obtained for each of the illuminations.

3. Method according to Claim 2, in which a measurement of the position of the pupil (6) of the eye is carried out, characterized in that with the eye being illuminated using a first light intensity, the step of creating the corrected image, on which the position of the pupil is measured, consists in superposing the images (I1, I3) obtained for each of the illuminations.

4. Method according to Claim 2, in which a measurement of the position of the corneal reflection (7) of the eye is carried out, characterized in that with the eye being illuminated using a second light intensity, the step of creating the corrected image consists in choosing the image (I2, I4) whose direction of illumination is closest angularly to the corneal reflection.

5. Method according to Claims 3 and 4, in which the positions of the pupil and of the corneal reflection are both measured, characterized in that it consists in illuminating the eye using an illumination sequence in which the eye is illuminated successively in one or other of the directions, or both.

6. Device for measuring the movements of an eye, implementing the method according to any one of Claims 1 to 5, including:
- means (12a, 12b) for illuminating the eye in at least two distinct directions; and
- video means (11) for creating images of the eye upon each illumination,
characterized in that it additionally includes processing means (20) for creating an image corrected for the reflections due to each illumination, by combination of the said at least two images, and for determining the movements of the eye from the position of the centre of gravity of the said eye on this corrected image.

7. Device according to Claim 6, characterized in that the means for illuminating the eye include at least two light sources oriented differently in relation to the eye, in such a way that the reflections on the eye relative to the illumination in the distinct directions are dissociated from one another.

8. Device according to Claim 7, characterized in that the means for illuminating the eye additionally include a current generator (30) connected to each of the light sources, to which it delivers a current of variable strength and/or duration, providing for illuminations of diverse light intensities.

9. Device according to Claim 7 or 8, characterized in that the light sources each comprise a plurality of synchronized light-emitting diodes, each diode being arranged in a particular orientation with respect to the eye.

10. Device according to any one of Claims 6 to 9, characterized in that the processing means include an analog/digital converter (22) for transforming the images obtained by the video means into binary images, the measurement of the movements of the eye being carried out on these binary images.

11. Device according to Claim 8, characterized in that the processing means include means (24) of combination for creating a corrected image by superposition of the images obtained upon alternating illumination by the light sources.

12. Device according to Claim 11, characterized in that the means of combination include a memory (26) connected to an output of the analog/digital converter and a logic OR circuit (28) connected, on the one hand, to the output of the said converter and, on the other hand, to an output of the memory and supplying the corrected image at the output.

13. Device according to any one of Claims 6 to 12, characterized in that it additionally includes a sequencer (32) for synchronizing the video means, the means for illuminating the eye, and the processing means.

## Patentansprüche

1. Verfahren zur Messung der Augenbewegungen, die folgenden Schritte umfassend:
- Beleuchten des Auges (9) aus wenigstens zwei verschiedenen Richtungen;
- Erfassen von wenigstens zwei Bildern des derart beleuchteten Auges (I1, I2, I3, ...)
- wenn diese Bilder des Auges erfaßt sind:
. Umsetzen jedes erfaßten Bildes in ein binäres Bild,
. Herstellen eines korrigierten Bildes (IC1) aus allen beleuchtungsbedingten Reflexen auf dem Auge durch Kombination der Binärbilder;
- Festlegen, auf dem korrigierten Bild, der Augenstellung durch Berechnung des Schwerpunkts des besagten Auges.

2. Verfahren nach Anspruch 1, dadurch gekennzeichnet, daß der Augenbeleuchtungsschritt darin besteht, das Auge wechselweise aus der einen und der anderen Richtung zu beleuchten, wobei bei jeder dieser Beleuchtungen ein Bild des Auges erfaßt wird.

3. Verfahren nach Anspruch 2, in dem man eine Messung der Stellung der Pupille des Auges durchführt, dadurch gekennzeichnet, wobei das Auge mit einer ersten Lichtintensität beleuchtet wird, daß der Schritt zur Herstellung des korrigierten Bildes, in dem die Pupillenstellung gemessen wird, darin besteht, die bei jeder der Beleuchtungen erhaltenen Bilder (I1, I3) zu überlagern.

4. Verfahren nach Anspruch 2, in dem man eine Messung der Position des Hornhaut- bzw. Kornealreflexes (reflet cornéen) (7) des Auges durchführt, dadurch gekennzeichnet, wobei das Auge mit einer zweiten Lichtintensität beleuchtet wird, daß der Schritt zur Herstellung des korrigierten Bildes darin besteht, das Bild (I1, I4) auszuwählen, dessen Beleuchtungsrichtung winkelmäßig dem Kornealreflex am nächsten kommt.

5. Verfahren nach den Ansprüchen 3 und 4, bei dem man zugleich die Messung der Stellungen der Pupille und des Kornealreflexes durchführt, dadurch gekennzeichnet, daß es darin besteht, das Auge entsprechend einer Beleuchtungsfolge zu beleuchten, in der das Auge sukzessive aus der einen oder der anderen oder den beiden Richtungen beleuchtet wird.

6. Vorrichtung zur Messung der Augenbewegungen mittels Anwendung des Verfahrens nach einem der Ansprüche 1 bis 5, umfassend:
- Einrichtungen (12a, 12b) zur Beleuchtung des Auges aus wenigstens zwei verschiedenen Richtungen; und
- Videoeinrichtungen (11), um bei jeder Beleuchtung Bilder des Auges herzustellen,
**dadurch gekennzeichnet,**
daß sie außerdem Verarbeitungseinrichtungen (20) umfaßt, um ein korrigiertes Bild der durch jede Beleuchtung hervorgerufenen Reflexe herzustellen, durch Kombinieren der genannten Bilder, wenigstens zwei, und die Augenbewegungen zu bestimmen aufgrund der Lage des Schwerpunkts des besagten Auges in diesem korrigierten Bild.

7. Vorrichtung nach Anspruch 6, dadurch gekennzeichnet, daß die Beleuchtungseinrichtungen des Auges wenigstens zwei Lichtquellen umfassen, die in bezug auf das Auge unterschiedlich ausgerichtet sind, so daß die die verschiedenen Beleuchtungsrichtungen betreffenden Reflexe auf dem Auge voneinander getrennt sind.

8. Vorrichtung nach Anspruch 7, dadurch gekennzeichnet, daß die Beleuchtungseinrichtungen des Auges außerdem einen Stromgenerator (30) umfassen, verbunden mit jeder der Lichtquellen, an die er einen Strom von variabler Stärke und/oder Dauer liefert, um Beleuchtungen diverser Lichtstärken zu gewährleisten.

9. Vorrichtung nach Anspruch 7 oder 8, dadurch gekennzeichnet, daß jede der Lichtquellen eine Vielzahl synchronisierter Lumineszenzdioden enthält, wobei jede Diode bezüglich des Auges entsprechend einer speziellen Ausrichtung angeordnet ist.

10. Vorrichtung nach einem der Ansprüche 6 bis 9, dadurch gekennzeichnet, daß die Verarbeitungseinrichtungen einen Analog-Digital-Wandler (22) umfassen, um die durch die Videoeinrichtungen erhaltenen Bilder in Binärbilder umzuwandeln, wobei die Messung der Augenbewegungen an diesen Binärbildern durchgeführt wird.

11. Vorrichtung nach Anspruch 8, dadurch gekennzeichnet, daß die Verarbeitungseinrichtungen Kombinationseinrichtungen (24) umfassen, um ein korrigiertes Bild herzustellen mittels Überlagerung der Bilder, die während der abwechselnden Beleuchtungen durch die Lichtquellen erhaltenen wurden.

12. Vorrichtung nach Anspruch 11, dadurch gekennzeichnet, daß die Kombinationseinrichtungen einen Speicher (26) enthalten, verbunden mit einem Ausgang des Analog-Digital-Wandlers und einer logischen ODER-Schaltung (28), einerseits verbunden mit dem Ausgang des Wandlers, andererseits mit einem Ausgang des Speichers und am Ausgang das korrigierte Bild liefernd.

13. Vorrichtung nach einem der Ansprüche 6 bis 12, dadurch gekennzeichnet, daß sie außerdem eine Folgeschaltung (32) enthält, um die Videoeinrichtungen, die Augenbeleuchtungseinrichtungen und die Verarbeitungseinrichtungen zu synchronisieren.
